# EUROPEAN PATENT SPECIFICATION

(11) **EP 2 840 062 B1**
(45) Date of publication and mention of the grant of the patent: **04.09.2019**
(21) Application number: 13777574.8
(22) Date of filing: 10.04.2013
(51) Int. Cl.: C01B 7/14, C07C 17/25, C07C 17/275, C07C 19/16, C07C 21/18

(54) **METHOD FOR UTILIZING FLUOROALKYL IODIDE**
VERFAHREN ZUR VERWENDUNG VON FLUORALKYLIODID
PROCÉDÉ D'UTILISATION D'IODURE DE FLUOROALKYLE

(30) Priority: 19.04.2012 JP 2012095432
(43) Date of publication of application: 25.02.2015
(73) Proprietor: Daikin Industries, Ltd., Osaka-shi, Osaka 530-8323 (JP)
(72) Inventor: SHIMADA, Tomoo, Osaka-shi Osaka 530-8323 (JP); HIRASAKA, Takeomi, Osaka-shi Osaka 530-8323 (JP); KUME, Takuji, Osaka-shi Osaka 530-8323 (JP)
(74) Representative: Hoffmann Eitle
(86) International application number: PCT/JP2013/060791
(87) International publication number: WO 2013/157456

(56) References cited:
- WO-A1-03/106023
- JP-A- 2004 035 302
- JP-A- 2004 035 302
- JP-A- 2004 269 413
- JP-A- 2004 269 413
- JP-A- 2010 030 965
- JP-A- 2010 030 965
- US-A1- 2005 250 966
- RONDESTVEDT C S ET AL: "Methyl-terminated perfluoroalkyl iodides and related compounds", THE JOURNAL OF ORGANIC CHEMISTRY, AMERICAN CHEMICAL SOCIETY, US, vol. 42, no. 11, 1 January 1977 (1977-01-01), pages 1985-1990, XP002369812, ISSN: 0022-3263, DOI: 10.1021/JO00431A034
- NEAL O BRACE ET AL: "Effect of a perfluoroalkyl group on the elimination and substitution reactions of two homologous series of perfluoroalkyl-substituted iodoalkanes", THE JOURNAL OF ORGANIC CHEMISTRY, AMERICAN CHEMICAL SOCIETY, US, vol. 49, no. 13, 1 January 1984 (1984-01-01), pages 2361-2368, XP002714924, ISSN: 0022-3263
- NEAL 0. BRACE ET AL.: 'Effect of a Perfluoroalkyl Group on the Elimination and Substitution Reactions of Two Homologous Series of Perfluoroalkyl-Substituted Iodoalkanes' J. ORG. CHEM. vol. 49, 1984, pages 2361 - 2368, XP002714924
- PAWAR, RAMESH R. ET AL: "Solubility and Density of Potassium Iodide in Binary Ethanol-Water Solvent Mixture at (298.15, 303.15, 308.15, and 313.15) K", JOURNAL OF CHEMICAL & ENGINEERING DATA , 54(6), 1935-1937 CODEN: JCEAAX; ISSN: 0021-9568, 2009, DOI: 10.1021/JE800682P 10.1021/JE800682P
- PAWAR, RAMESH R. ET AL: "Solubility and density of potassium iodide in binary ethanol-water solvent mixture at (298.15, 303.15, 308.15, and 313.15) K. [Erratum to document cited in CA150:549108]", JOURNAL OF CHEMICAL & ENGINEERING DATA , 55(2), 1073 CODEN: JCEAAX; ISSN: 0021-9568, 2010, DOI: 10.1021/JE900848F 10.1021/JE900848F

## Description

### Technical Field

The present invention relates to a method for utilizing a fluoroalkyl iodide, and, more specifically, to a method for effectively utilizing a fluoroalkyl iodide that has an unnecessary chain length by recovering an alkali metal iodide or solid iodine from the fluoroalkyl iodide obtained by a telomerization reaction.

### Background Art

Fluoroalkyl iodide is a compound that is extremely useful as a functional material, such as an oil- and water-repellant agent or an emulsifier; or as an intermediate in organic synthesis. Fluoroalkyl iodide is produced mainly by a telomerization reaction using tetrafluoroethylene as a starting material.

A telomerization reaction is a reaction for obtaining a polymer having a low polymerization degree, namely, a telomer, by adding taxogen to telogen using a thermal or optical effect in the presence of an appropriate catalyst.

However, since telomerization reaction is a type of polymerization reaction, the crude products from a telomerization reaction always have a distribution in the carbon number. Therefore, the fluoroalkyl iodide telomers obtained by a telomerization reaction always include a certain amount of long-chain telomers, which have less value in industrial use. Thus, these long-chain telomers need to be recycled in view of, for example, efficiently using precious resources and reducing production costs.

As a method for recycling long-chain telomers, a method that involves mixing a fluoroalkyl iodide having 6 or more carbon atoms with a fluoroalkyl iodide having 4 or fewer carbon atoms, and performing a heat treatment of the mixture, thereby obtaining a fluoroalkyl iodide having 4-6 carbon atoms, has been reported (see WO 2011/256312). However, this method has low selectivity for the target short-chain fluoroalkyl iodide, and produces a telomer compound having a distribution in the carbon number, as well as e.g. perfluorocarbon and iodine as by-products. Therefore, this method requires a process for isolating and purifying the desired fluoroalkyl iodide. However, the process for isolating and purifying the desired fluoroalkyl iodide is complicated because the products also include perfluorocarbons, which also have a distribution in the carbon number, as does the telomer compound. Further, iodine and perfluorocarbons having a large number of carbon atoms (in particular, 12 or more carbon atoms) are solid at room temperature; therefore, they solidify in production facilities and may cause blockage of devices and pipes. Moreover, under the temperature range of 450-495°C, which is considered to be an appropriate range for this method, iodine has significantly remarkable corrosive properties with respect to metal materials. This raises a concern regarding the maintenance of the production facilities.

Further, a method of reacting a fluoroalkyl iodide with hydrogen to convert the iodide (Rf-I) into a hydride (Rf-H), and then recovering and recycling iodine or potassium iodide has also been suggested (see US 6,525,231). However, since this method requires handling iodine and hydrogen iodide under high temperatures, as in the aforementioned heat decomposition of telomers, it raises a problem of reducing the lifespan of the production facilities. Further, if the reaction temperature is decreased to suppress the generation of perfluorocarbon due to a side reaction, or the decomposition of perfluoroalkyl chains, the conversion rate in the reaction will decrease. As a result, a process for isolating and purifying the target compound will be necessary, thus complicating the manufacture.

On the other hand, a method of reacting a perfluoroalkyl iodide and an alkali metal hydroxide in the presence of methanol, thereby obtaining a 1H-perfluoroalkane and an alkali metal iodide, has also been reported (see JP-B-2559312). Although this method is capable of converting a perfluoroalkyl iodide into a 1H-perfluoroalkane and an alkali metal iodide, the method requires a large excess of alkali metal hydroxide relative to the perfluoroalkyl iodide; hence, the alkali metal iodide recovered after the reaction is obtained as a mixture containing a large amount of alkali metal hydroxide. Therefore, an isolation and purification step will be necessary for the subsequent recovering and recycling. As such, this method can hardly be considered efficient. Further, when a fluoroalkyl iodide and an alkali metal hydroxide are reacted by this method, formaldehyde is produced as a by-product together with 1H-perfluoroalkane due to proton donation from methanol. Therefore, in this method, methanol is consumed for each batch, and thus needs to be added each time; further, an isolation and purification step is necessary when methanol is recovered and recycled after the reaction. Moreover, since formaldehyde is an easily polymerizable substance, it may be polymerized within the production facilities, and cause blockage or contamination of the facilities.

C.S. Rondestvedt, Jr., J. Org. Chem., 42(11), 1985-90 (1977) reports on the preparation of methyl-terminated perfluoroalkyl iodides and related compounds. The disclosed reaction scheme includes the reaction of CH₂=CF₂ with HI to form CH₃CF₂I (2), the reaction thereof with C₂F₄ to form CH₃(CF₂)*ₙ*I (3) and the further conversion of this intermediate with C₂H₄ to obtain CH₃(CF₂)*ₙ*CH₂CH₂I (4). The latter is then treated with a base to obtain CH₃(CF₂)*ₙ*CH=CH₂ (9).

### Summary of Invention

### Technical Problem

The present invention has been accomplished in view of the foregoing state of the art, and its primary object is to provide a method for effectively reusing telomers that have an unnecessary chain length, in particular, long-chain telomers, that are formed when a fluoroalkyl iodide is produced by a telomerization reaction, without requiring any complicated separation procedure, and without adversely affecting equipment.

### Solution to Problem

The present inventors conducted extensive research to achieve the above object. As a result, the inventors found that, according to a method comprising using fluoroalkyl iodides with various chain lengths as objects to be treated, adding ethylene to each fluoroalkyl iodide to prepare an ethylene adduct thereof, reacting the adduct with an alkali metal hydroxide and/or an alkali metal alkoxide in the presence of a lower alcohol to perform a dehydroiodination reaction to thereby form an olefin compound, iodine atom of the fluoroalkyl iodide can be recovered as an alcohol solution of an alkali metal iodide. According to this method, the ethylene addition reaction and the dehydroiodination reaction both proceed with very high conversion and selectivity; therefore, an alkali metal iodide can be recovered in a very high yield. In addition, since the dehydroiodination reaction does not require a large excess of alkali metal hydroxide, the alkali metal iodide obtained after the reaction have sufficiently high purity, and can be easily reproduced as iodine without requiring a complicated treatment. Further, this method does not require handling e.g. iodine and hydrogen iodide at a high temperature, and thus can solve problems such as corrosion and blockage of facilities. Moreover, in the case of using methanol as the lower alcohol, used methanol can be easily recovered at high purity from a methanol solution of an alkali metal iodide, for example, by simple distillation, since water, the only by-product in the olefination reaction, is non-azeotropic with methanol. The recovered methanol can be reused as a solvent. The present invention has been accomplished based on these novel findings.

More specifically, the present invention provides a method comprising the steps of:
(i) reacting ethylene with the fluoroalkyl iodide of the formula Rf(CF₂CF₂)*ₙ*I (1) to prepare an ethylene adduct of the formula Rf(CF₂CF₂)*ₙ*CH₂CH₂I (2) ;
(ii) reacting the obtained ethylene adduct of formula (2) with at least one compound selected from lithium hydroxide, sodium hydroxide, lithium methoxide, lithium ethoxide, sodium methoxide and sodium ethoxide, in the presence of a lower alcohol and in the presence of ≤ 11.5 part by volume of water per 100 parts by volume of alcohol, thus preparing, by dehydroiodination, an olefin compound of the formula Rf(CF₂CF₂)*ₙ*CH=CH₂ (3) and an alkali metal iodide; and
(iii) collecting the alkali metal iodide;
wherein in formulae (1)-(3) Rf is C₁₋₁₀-fluoroalkyl and n is an integer of ≥ 1.

Preferred embodiments of the present invention are as defined in the appended dependent claims and/or in the following detailed description. The method of the present invention (referred to as the present method hereinafter) is described in detail below.

### (1) Object to be treated

The object to be treated by the present method is a fluoroalkyl iodide of formula (1) : R_{f}(CF₂CF₂)ₙI, wherein R_{f} is C₁₋₁₀ fluoroalkyl and n is an integer of ≥ 1.

The fluoroalkyl iodide of formula (1) above can be obtained, for example, by a telomerization reaction of a fluoroalkyl iodide represented by R_{f}-I with tetrafluoroethylene. In R_{f}-I, R_{f} is C₁₋₁₀ fluoroalkyl, preferably C₁₋₈ fluoroalkyl, and more preferably C₁₋₅ fluoroalkyl. It is preferable that R_{f} be perfluoroalkyl.

Specific examples of R_{f}-I include trifluoromethyl iodide, pentafluoroethyl iodide, perfluoroisopropyl iodide and perfluoro-n-butyl iodide. Of these, pentafluoroethyl iodide is often used in a telomerization reaction with tetrafluoroethylene.

In a fluoroalkyl iodide telomer of formula (1) to be treated by the present method, polymerization degree n is an integer of ≥ 1, and generally in the range of 2-12. For example, a long-chain telomer having a carbon number of ≥ 8 is also effectively treated. Generally, in the present method, a mixture of fluoroalkyl iodide telomers having different values of polymerization degree n can also be treated.

### (2) Treatment method

In the present method, a fluoroalkyl iodide of formula (1) above (the object to be treated) is reacted with ethylene to prepare an ethylene adduct. Thereafter, the ethylene adduct is reacted with at least one alkali metal compound selected from lithium hydroxide, sodium hydroxide, lithium methoxide, lithium ethoxide, sodium methoxide and sodium ethoxide in the presence of a lower alcohol, and in the presence of ≤ 11.5 part by volume of water per 100 parts by volume of alcohol, to cause a dehydroiodination reaction to thereby prepare an olefin compound. In this manner, the iodine atom in the fluoroalkyl iodide can be recovered as an alcohol solution of an alkali metal iodide.

The alkali metal iodide recovered by the method above is useful as a starting material for various chemical reactions. Further, when the alkali metal iodide recovered by the method above is treated by a method described below, it is possible to easily obtain solid iodine, which is an important substance, for example, as a starting material or intermediate for various products, such as medical, industrial, and agricultural products.

Each step of the present method is described in detail below.

### (i) Ethylene addition step

In the ethylene addition step, ethylene is added to a fluoroalkyl iodide of the formula R_{f}(CF₂CF₂)ₙI (1), wherein Rf is C₁₋₁₀ fluoroalkyl and n is an integer of ≥ 1, to prepare an ethylene adduct of the formula R_{f}(CF₂CF₂)ₙCH₂CH₂I (2), wherein Rf and n are the same as above.

This step can be performed under conditions generally employed in ethylene addition reactions. More specifically, ethylene can be added at a reaction temperature of 50-200°C (e.g., 70-120°C) and at a reaction pressure of 0.01-3 MPa (e.g., 0.1-1 MPa). The reaction time is generally 0.5-4 hours. The reaction pressure is the pressure generated by the ethylene introduced.

The amount of ethylene is preferably 1-1.2 moles per mole of the fluoroalkyl iodide of formula (1).

The ethylene addition reaction is preferably performed in the presence of a radical-generating catalyst. Usable catalysts are, for example, azo compounds and organic peroxides. Examples of azo compounds include α,α'-azobisisobutyronitrile. Examples of organic peroxides include diacyl peroxides, such as benzoyl peroxide; dialkyl peroxides, such as t-butyl peroxide; and peroxymonocarbonates, such as t-butylperoxy isopropyl carbonate. The amount of the catalyst is preferably 0.005-0.02 moles per mole of the fluoroalkyl iodide of formula (1).

Moreover, when the ethylene addition reaction is performed in the presence of a metallic copper catalyst, high conversion can be achieved, and a monoethylene adduct can be produced with high selectivity. Furthermore, the metallic copper catalyst, which is a solid catalyst, has the advantage that it can be readily separated from the reaction product and reused.

The metallic copper catalyst is not particularly limited as long as it is copper elementary metal. In view of catalytic activity, the form of the metallic copper catalyst is preferably powdery, which ensures a large contact area of the metallic copper with the starting materials. The average particle size of the powdery copper is, for example, 0.1-300 µm, and preferably 30-150 µm. The average particle size of the powdery copper is measured by a laser diffraction-type particle-size-distribution measuring apparatus (e.g., MICROTRAC HRA Model No. 9320-X100, produced by Nikkiso Co., Ltd.). The amount of metallic copper catalyst used is, for example, 0.1-90 parts by weight (pbw), and preferably 0.5-10 pbw, based on 100 pbw of the fluoroalkyl iodide of formula (1).

The metallic copper catalyst may be a catalyst in which metallic copper is supported on a carrier. Usable carriers are not particularly limited as long as the activity of the metallic copper catalyst is not adversely affected, and examples thereof include metal oxides. Specific examples are single-metal oxides selected from zinc oxide, iron oxide, copper oxide, titanium oxide, zirconium oxide, cerium oxide, aluminum oxide, and silicon oxide; and complex oxides comprising two or more metals selected from zinc, iron, copper, titanium, zirconium, cerium, aluminum, and silicon. Although the form of the carrier that supports metallic copper is not particularly limited, a powdery carrier is preferable in view of catalytic activity. The metallic copper can be supported on the carrier by a known method.

The amount of metallic copper in the metallic copper catalyst wherein the metallic copper is supported on a carrier is 0.01-50 wt.%, and preferably 0.1-20 wt.%, based on the total amount (100 wt.%) of the catalyst.

The amount of the metallic copper catalyst wherein metallic copper is supported on a carrier is, for example, 0.1 to 90 pbw, and preferably 0.5-10 pbw, based on 100 pbw of the fluoroalkyl iodide of formula (1).

Further, other metals may be added to the metallic copper catalyst so as to enhance the activity of the catalyst. Examples thereof include titanium, chromium, iron, cobalt, nickel and tin. Among these, tin is preferable. The amount of other metal used is, for example, 0.1-90 pbw, and preferably 10-30 pbw, based on 100 pbw of the metallic copper catalyst. The other metal is preferably powdery in view of catalytic activity.

In the ethylene addition reaction using a metallic copper catalyst, the fluoroalkyl iodide of formula (1) is reacted with ethylene under ethylene gas pressure in the presence of the metallic copper catalyst. The ethylene gas pressure is, for example, 0.01-3 MPa, and preferably 0.1-1 MPa. The amount of ethylene gas used is, for example, 1-1.2 moles per mole of the fluoroalkyl iodide of formula (1).

The ethylene addition reaction can be performed, for example, as follows: The fluoroalkyl iodide of formula (1) and optionally a metallic copper catalyst are placed in a pressure vessel for heating, such as an autoclave, and the vessel is deaerated. After the temperature is raised to the reaction temperature by a heater, ethylene gas is introduced into the vessel, and stirring is performed at that temperature for a specific period of time. The reaction temperature is, for example, 50-200°C, and preferably 70-120°C, in view of safety and the reaction rate.

In the ethylene addition reaction, as ethylene is consumed, the internal pressure of the reaction vessel decreases, which reduces the reaction rate. It is thus preferable to keep the internal pressure constant by successively supplying ethylene.

Although the reaction time of the ethylene addition reaction varies depending on the reaction conditions, it is generally 0.5-4 hours. The point where a decrease in the pressure of the ethylene is no longer observed can be determined to be the end point of the reaction.

### (ii) Dehydroiodination step

In the dehydroiodination step, the ethylene adduct of the formula R_{f}(CF₂CF₂)ₙCH₂CH₂I (2), wherein Rf and n are the same as above, obtained in step (i) is reacted with at least one alkali metal compound selected from lithium hydroxide, sodium hydroxide, lithium methoxide, lithium ethoxide, sodium methoxide and sodium ethoxide in the presence of a lower alcohol, and in the presence of ≤ 11.5 part by volume of water per 100 parts by volume of alcohol, to prepare, by dehydroiodination, an olefin compound of the formula R_{f}(CF₂CF₂)ₙCH=CH₂ (3), wherein Rf and n are the same as above. As a result, iodine contained in the ethylene adduct of formula (2) can be obtained as an alkali metal iodide.

Among the alkali metal compounds listed above, sodium hydroxide is particularly preferable in terms of availability at low cost, and also sodium methoxide and sodium ethoxide, which are relatively inexpensive and widely available commercially, are preferable.

The alkali metal compounds can be used singly, or in a combination of two or more.

The total amount of the at least one alkali metal compound is preferably 1-1.5 moles, and more preferably 1-1.2 moles, per mole of the ethylene adduct of formula (2).

The dehydroiodination reaction must be performed in the presence of a lower alcohol. The lower alcohol to be used preferably has 1-4 carbon atoms. Methanol and ethanol are particularly preferable. Methanol is particularly preferable because it is available at low cost, and can be easily recovered because it is non-azeotropic with water; and because only a small amount of olefin compound dissolved, resulting in a high yield after liquid-liquid separation. Lower alcohols can be used singly, or in a combination of two or more.

The amount of lower alcohol used is preferably such that the total amount of the at least one alkali metal compound falls within the range of 1.5-4 moles, more preferably 2-3 moles, per liter of the lower alcohol. When the lower alcohol is used in an amount such that the concentration of the alkali metal compound falls within the aforementioned specific range, an olefin compound of the formula R_{f}(CF₂CF₂)ₙCH=CH₂ (3), wherein Rf and n are the same as above, which is obtained after the reaction, is separated from the alcohol, allowing the reaction solution to be clearly separated into two layers, i.e., an olefin compound layer and an alcohol layer. The alkali metal iodide obtained by olefination reaction is contained in the alcohol layer.

The dehydroiodination reaction may be performed in the presence of water. However, an excessively large amount of water relative to the lower alcohol results in a reduced reaction rate, and might adversely affect the recovery of the lower alcohol after the reaction. Accordingly, water is used in an amount of ≤ 11.5 parts by volume per 100 parts by volume of the lower alcohol.

Although the method for carrying out the dehydroiodination reaction is not particularly limited, one example of the treatment method is as follows.

First, at least one alkali metal compound selected from lithium hydroxide, sodium hydroxide, lithium methoxide, lithium ethoxide, sodium methoxide and sodium ethoxide, a lower alcohol, and an ethylene adduct of formula (2) are introduced into a reaction vessel and reacted. During the reaction, the reaction solution may be heated or stirred, if necessary.

The at least one alkali metal compound may be used by adding the alkali metal compound to a lower alcohol simultaneously with an ethylene adduct, or dissolving the alkali metal compound in a lower alcohol before the addition of an ethylene adduct.

Although the reaction temperature is not particularly limited, the temperature is generally 0-200°C, preferably 20-160°C, from the viewpoints of the reaction rate, safety, and economic efficiency.

The pressure during the reaction is not particularly limited. The reaction can generally be performed under atmospheric pressure or increased pressure.

The reaction time may generally be 1-10 hours.

The reaction solution obtained by the above method is allowed to stand to separate the solution into two layers, i.e., a layer containing an olefin compound, and an alcohol layer. Because the target alkali metal iodide is contained in the upper alcohol layer, alkali metal iodide can be collected by using known methods, such as distillation and crystallization after collecting the alcohol layer by liquid-liquid separation.

The alkali metal iodide obtained in this step can be effectively used, for example, as a starting material for various chemical reactions.

Further, the alkali metal iodide obtained in this step has high purity and therefore can be recovered as high-purity iodine by a method described below, without requiring any complicated isolation and purification procedure.

### (iii) Iodine formation step

In the present invention, after the alkali metal iodide is obtained by the above method, iodine formation treatment may be performed to separate solid iodine and collect the solid iodine, thereby obtaining high-purity solid iodine.

The iodine formation treatment method is not particularly limited, and known methods can be suitably used. For example, an aqueous solution of the alkali metal iodide obtained in step (ii) is prepared, iodine is separate from the alkali metal iodide, and then solid iodine is isolated and collected. In a specific example of the methods, the alkali metal iodide obtained in step (ii) is formed into an aqueous solution, and the aqueous solution is treated with chlorine to separate iodine, thus obtaining an iodine slurry, after which iodine is isolated from the slurry. For example, after washing the slurry with water, solid iodine can be isolated by drying.

In the present invention, it is particularly preferable to use a method in which, after the iodine slurry is obtained by the above method, iodine in the slurry is melted by heating to induce liquid-liquid separation to thereby form two layers of water and molten iodine, and the molten iodine is supplied to a cooling device to solidify the molten iodine by cooling, and collect solid iodine.

In this method, the concentration of the alkali metal iodide in the aqueous solution may be, for example, 10-40 wt%, and preferably 20-30 wt%. An overly low concentration increases the volume of water, requiring a large reaction vessel, and increasing the ratio of iodine dissolved in the aqueous solution, which decreases the amount of iodine collected. The pH of the aqueous solution is preferably adjusted to 4-6 to inhibit iodic acid generation.

An aqueous solution containing an alkali metal iodide and chlorine gas can generally be reacted by introducing the chlorine gas into the aqueous solution. For example, a method of bubbling chlorine gas in the aqueous solution can be used. This reaction is preferably performed in a sealed system to avoid toxic chlorine gas diffusion. Although the supply rate of the chlorine gas varies depending on the reaction scale, it may be, for example, 10-150 kg/hr. The reaction temperature may be, for example, 10-80°C. The reaction pressure may generally be about normal pressure. Although the reaction time varies depending on the reaction conditions, particularly the supply rate of chlorine gas, it is, for example, 0.1-10 hours. When the supply amount of the chlorine gas is less than one equivalent relative to one equivalent of alkali metal iodide in water, unreacted alkali metal iodide remains. Conversely, an overly large chlorine gas supply amount generates, for example, iodic acid. Therefore, for example, a method of adjusting an appropriate reaction time by monitoring the oxidation-reduction potential can be employed.

After the reaction is completed, the iodine slurry settles to the bottom of the treatment vessel used for chlorination treatment.

The iodine slurry thus obtained is supplied to an iodine melting vessel. In the iodine melting vessel, to improve the purity of iodine to be collected, heating is performed at 120-140°C to melt iodine in the slurry to induce liquid-liquid separation. By this process, an aqueous layer and a molten iodine layer are respectively separated into an upper layer and a lower layer. The iodine melting vessel is preferably a sealed system, and the pressure during melting may generally be 0.15-0.2 MPa.

Subsequently, the molten iodine present in the bottom portion of the iodine melting vessel is supplied to a cooling device, and solidified by cooling to collect iodine as solid iodine.

In this case, in place of a method of directly cooling and solidifying the molten iodine present in the bottom portion of the iodine melting vessel, the molten iodine may be subjected to the following steps and then solidified by cooling. The purity of the resulting solid iodine can thereby be improved.

In this method, first, the molten iodine present in the bottom portion of the iodine melting vessel is transferred to a washing vessel for the molten iodine. For example, the molten iodine may be added dropwise to water stirred in the washing vessel. Thus, solid iodine having an average particle size of about 2-3 mm is obtained, and the solid iodine settles in the washing vessel. The water temperature is preferably 20-30°C. The washing vessel is preferably a sealed system, and the pressure is generally about normal pressure. In the washing vessel, metal ions, such as an extremely small amount of potassium ions contained in the molten iodine, are removed by washing the molten iodine with water.

The solid iodine and water in the washing vessel are supplied into the iodine melting vessel, and separated into two layers of water and molten iodine. The temperature in the iodine melting vessel is generally kept at 120-140°C. The iodine melting vessel is preferably a sealed system, and the pressure is generally 0.15-0.2 MPa.

The molten iodine obtained in this step is supplied to a cooling device, and solidified by cooling, thus obtaining solid iodine having higher purity.

The cooling device may be a device capable of rapidly solidifying the supplied molten iodine by cooling; for example, a rotation drum having a cooling system can be used. Examples of the rotation drum having a cooling system include a rotation drum in which a refrigerant such as water is circulated in the drum. The material of the rotation drum is not particularly limited as long as the rotation drum is stable at a molten iodine temperature, and resists corrosion by e.g. iodine. For example, corrosion- and heat-resistant alloys such as Hastelloy C22 can be used. The shape of the rotation drum may be, for example, cylindrical or disk-shaped. The outer diameter of the rotation drum may be 300-500 mm. Although the rotation rate of the rotation drum varies depending on the supply rate of molten iodine, it may be, for example, 4-7 rpm.

The molten iodine supply portion and the cooling device are preferably positioned adjacent to each other so that the molten iodine is supplied from e.g. a nozzle, and rapidly solidified by cooling on the rotation drum. When they are overly apart from each other, iodine to be sublimated is increased, reducing the yield of solid iodine; therefore, this is not preferable.

The solid iodine solidified by cooling using the cooling device is removed by scraping using a scraping device, and stored as flake iodine in a collection container. Although the scraping device is not particularly limited, when a rotation drum is used as a cooling device, for example a squeegee or a scraping blade capable of scraping iodine solidified by cooling on the rotation drum can be used.

The flow path through which the molten iodine passes and the molten iodine supply portion are preferably heated without interruption at the iodine melting temperature (113°C) or more so that the iodine does not solidify midstream.

By using the iodine collecting method mentioned above, solid iodine can be recovered in a yield of ≥ 98% from an aqueous solution containing an alkali metal iodide.

In collecting molten iodine as solid iodine by the above-described method, the molten iodine supply portion, cooling device, scraping device, and collection container for solid iodine are preferably installed in a sealed chamber to prevent emission of sublimated iodine.

The sublimated iodine generated in such a sealed chamber may be dissolved in an aqueous solution containing an alkali metal iodide, and treated with chlorine in the same manner as described above to separate iodine, thereby giving an iodine slurry. The iodine slurry is heated to melt the iodine, and then the molten iodine is supplied to a cooling device to solidify the molten iodine by cooling. With this method, the sublimated iodine is recovered as solid iodine.

For dissolving sublimated iodine in an aqueous solution containing an alkali metal iodide, for example, a method comprising bubbling a sublimated iodine-containing gas in an alkali metal iodide aqueous solution, and a method comprising passing a sublimated iodine-containing gas through a container in which an alkali metal iodide aqueous solution is sprayed, can be used. In particular, the below-described method using a sublimated iodine absorption column achieves efficient absorption.

In this method, a gas containing sublimated iodine generated in a sealed chamber is passed through a sublimated iodine supply tube, and introduced, from a sublimated iodine inlet, into the sublimated iodine absorption column. The flow rate at which the sublimated iodine-containing gas is introduced into the absorption column is controlled by pump adjustment. Although the flow rate varies depending on the size of the sublimated iodine absorption column, it may generally be 1,000-1,500 m³/hr.

A specific example of the sublimated iodine absorption column is an absorption column packed with a packing material to increase the area of contact between the alkali metal iodide aqueous solution and the sublimated iodine. Examples of the packing material include glass packing materials and resin packing materials (e.g., polypropylene and polyethylene), which resist corrosion by iodine. At least one packing material selected from the group consisting of these examples is preferably used. The size and shape of the packing material and the method of placing the packing material are suitably selected so as to maintain the number of theoretical plates sufficient to increase the area of contact between the alkali metal iodide aqueous solution and the sublimated iodine, and thereby enhance the efficiency of sublimated iodine absorption. Examples of the size and shape of packing material include a bead shape having an outer diameter of about 10 mm and a Raschig ring having an outer diameter of about 10 mm. Among them, a Raschig ring is preferable. For packing with a packing material, a known method may be used.

Sublimated iodine is absorbed by a sublimated iodine absorption column, for example, as follows. From the inlet provided at the upper portion of the absorption column, an alkali metal iodide aqueous solution is introduced into the absorption column, and allowed to flow downward while coming into contact with the surface of a packing material. From the sublimated iodine inlet provided at the lower portion of the absorption column, a sublimated iodine-containing gas is introduced into the absorption column. Because the sublimated iodine-containing gas comes into contact with the alkali metal iodide aqueous solution that is flowing downward over the packing material, the sublimated iodine is absorbed (dissolved) into the alkali metal iodide aqueous solution. From the outlet provided at the lower portion of the absorption column, the alkali metal iodide aqueous solution that has absorbed iodine is discharged.

The alkali metal iodide aqueous solution used for the sublimated iodine absorption column preferably has a concentration at which effective iodine absorption occurs. For example, the concentration is 10-30 wt%, and preferably 20-30 wt%. The supply rate of the alkali metal iodide aqueous solution at the inlet is, for example, 1,000-3,000 L/hr, and preferably 2,000-3,000 L/hr.

The alkali metal iodide aqueous solution that has absorbed iodine is, after being discharged from the outlet of the sublimated iodine absorption column, passed through a tube outside the absorption column, and returns to the inlet at the upper portion of the absorption column. This enables effective absorption of sublimated iodine by the use of a constant amount of alkali metal iodide aqueous solution.

The sublimated iodine-absorbing alkali metal iodide aqueous solution obtained by the above method is treated with chlorine in the same manner as described above to separate iodine, thereby giving an iodine slurry. The iodine slurry is heated to melt the iodine, and the molten iodine is supplied to a cooling device to solidify the molten iodine by cooling. Accordingly, sublimated iodine is recovered as solid iodine. With this method, sublimated iodine generated in a sealed chamber is recovered as solid iodine in a yield of ≥ 96%.

The above-described method enables a high-yield recovery of solid iodine from fluoroalkyl iodide telomers that have an unnecessary chain length obtained by a telomerization reaction. The iodine recovered by this method can be effectively used as, for example, a starting material or intermediate for various products, such as medical, industrial, and agricultural products.

### Advantageous Effects of Invention

According to the present method, an alkali metal iodide can be recovered in a very high yield from telomers that have an unnecessary chain length contained in fluoroalkyl iodide obtained by a telomerization reaction. The alkali metal iodide obtained by this method is usable as a starting material for various chemical reactions, and is a very useful substance.

Further, since the dehydroiodination reaction does not require a large excess of alkali metal hydroxide, the obtained alkali metal iodide has high purity, and can be easily regenerated as solid iodine without requiring a complicated treatment. The solid iodine collected by this method is highly useful as, for example, a starting material or intermediate for various products, and is an expensive and valuable resource due to its low abundance.

Furthermore, the present method does not require handling iodine or hydrogen iodide at a high temperature; therefore, problems such as equipment corrosion and blockage do not occur. In addition, when methanol is used as the alcohol, it can be easily recovered at high purity from an alkali metal iodide alcohol solution, for example, by simple distillation, since water, the only by-product in the olefination reaction, is non-azeotropic with methanol, and the recovered methanol can be reused as a solvent.

Accordingly, the present method allows effective utilization of telomers that have an unnecessary chain length contained in fluoroalkyl iodide telomers obtained by a telomerization reaction, thus achieving very significant effects, such as efficiently using precious resources and reducing production costs.

### Description of Embodiments

In order to show the efficacy of each step of the present method, the present invention is described in detail below with reference to Examples of each step.

### Example 1: Ethylene addition step

130.8 g of 1-iodoperfluorooctane (n-C₈F₁₇I) and 2.66 g of copper powder were placed in a 200-mL Hastelloy autoclave, followed by deaeration. After heating to 80°C, ethylene was introduced thereinto, making the internal pressure of the autoclave 0.1 MPa. Ethylene was introduced into the autoclave while keeping the internal pressure at 0.1 MPa, and the point where a decrease in the pressure of the ethylene was no longer observed was determined to be the end point of the reaction. The reaction time was 120 minutes. After cooling the reaction mixture, gas chromatography analysis was performed to find that the conversion of the 1-iodoperfluorooctane was 99.7%, and the selectivity of the generated ethylene adduct (monoethylene adduct) was ≥ 99.9%.

### Example 2: Dehydroiodination step

89.7 mmol of 98% sodium hydroxide and 45 mL of methanol were placed in a 200-mL round-bottom flask equipped with a Dimroth condenser, followed by stirring at 50°C until the sodium hydroxide dissolved.

After dissolving the sodium hydroxide, 50.02 g of 1H,1H,2H,2H-1-iodoperfluorodecane was added thereto, followed by further stirring at 50°C for 2 hours.

After heating and stirring were stopped and the sample was allowed to stand, the two layers of the solution were separated, and 50.72 g of the upper layer was collected as a methanol solution of sodium iodide. After filtration using filter paper, 13.75 g of sodium iodide as a white solid was obtained by evaporating the solvent. X-ray powder diffraction analysis found that the result was a mixture of anhydrous sodium iodide and sodium iodide dihydrate.

### Example 3: Dehydroiodination step

29.9 g of 1H,1H,2H,2H-1-iodoperfluorodecane, 53.9 mmol of 98% sodium hydroxide, and 26.9 mL of methanol were placed in a 100-mL shaking-type autoclave, followed by stirring in an oil bath at 80°C for 2 hours.

After heating and stirring were stopped and the sample was allowed to stand, the two layers of the solution were separated, and 31.34 g of the upper layer was collected as a methanol solution of sodium iodide. After filtration using filter paper, 8.16 g of sodium iodide as a white solid was obtained by evaporating the solvent. X-ray powder diffraction analysis found that the result was a mixture of anhydrous sodium iodide and sodium iodide dihydrate.

### Example 4: Dehydroiodination step

108.8 mmol of 98% sodium hydroxide and 43.5 mL of methanol were placed in a 200 mL round-bottom flask equipped with a Dimroth condenser, followed by stirring at 50°C until the sodium hydroxide dissolved.

After dissolving the sodium hydroxide, 50.00 g of 1H,1H,2H,2H-1-iodoperfluorooctane was added thereto, followed by further stirring at 50°C for 2 hours.

After heating and stirring were stopped and the sample was allowed to stand, the two layers of the solution were separated, and 51.30 g of the upper layer was collected as a methanol solution of sodium iodide. After filtration using filter paper, 17.74 g of sodium iodide as a white solid was obtained by evaporating the solvent. X-ray powder diffraction analysis found that the result was a mixture of anhydrous sodium iodide and sodium iodide dihydrate.

From the results described above, it can be confirmed that in the olefination step of the method of the present invention, highly pure olefin can be obtained at high conversion using fluoroalkyl iodide telomers of various chain lengths.

### Example 5: Iodine recovery step

The sodium iodide obtained in the above step and the potassium iodide that was generated as a by-product in the substitution reaction of an organic iodine compound by a nucleophilic reagent were dissolved in water. After filtering the insoluble impurities under reduced pressure, a mixed aqueous solution of sodium iodide and potassium iodide was prepared. The concentration of each iodide in this aqueous solution was such that the concentration of sodium iodide was 11.02 wt% and that of potassium iodide was 11.44 wt%. A 4,944 kg amount of this aqueous solution was placed in a reaction vessel.

A 248 kg amount of chlorine gas was introduced into the aqueous solution at a reaction temperature of 10-60°C and at an introduction rate of 140 kg/hr to separate iodine, thereby obtaining an iodine slurry.

By washing the iodine slurry with water and then dehydrating it, 887 kg of solid iodine was obtained. The iodine recovery rate was 99.3%.

## Claims

1. A method comprising the steps of:
(i) reacting ethylene with the fluoroalkyl iodide of the formula Rf(CF₂CF₂)*ₙ*I (1) to prepare an ethylene adduct of the formula Rf(CF₂CF₂)*ₙ*CH₂CH₂I (2);
(ii) reacting the obtained ethylene adduct of formula (2) with at least one compound selected from lithium hydroxide, sodium hydroxide, lithium methoxide, lithium ethoxide, sodium methoxide and sodium ethoxide, in the presence of a lower alcohol and in the presence of ≤ 11.5 part by volume of water per 100 parts by volume of alcohol, thus preparing, by dehydroiodination, an olefin compound of the formula Rf(CF₂CF₂)*ₙ*CH=CH₂ (3) and an alkali metal iodide; and
(iii) collecting the alkali metal iodide;
wherein in formulae (1)-(3) Rf is C₁₋₁₀-fluoroalkyl and n is an integer of ≥ 1.

2. The method of claim 1, wherein the fluoroalkyl iodide of formula (1) has eight or more carbon atoms.

3. The method of claim 1 or 2, wherein the lower alcohol used in step (ii) is at least one of methanol and ethanol, the alkali metal hydroxide is sodium hydroxide, and the alkali metal alkoxide is at least one of sodium methoxide and sodium ethoxide.

4. The method of any of claims 1-3, wherein the obtained alkali metal iodide is further subjected to an iodine formation treatment to separate solid iodine, and collecting the solid iodine.

5. The method of claim 4, which comprises the steps of
(a) preparing an aqueous solution of the alkali metal iodide obtained in the method of any of claims 1-3;
(b) treating the solution with chlorine to separate iodine, thereby obtaining an iodine slurry;
(c) melting iodine in the slurry by heating to induce liquid-liquid separation to thereby form two layers of water and molten iodine;
(e) solidifying the molten iodine by cooling; and
(f) collecting solid iodine.

6. The method of claim 5, which further comprises between steps (c) and (e) a step of
(d) washing the molten iodine with water to give solid iodine and melting the solid iodine by reheating to induce liquid-liquid separation to thereby form two layers of water and molten iodine.

## Patentansprüche

1. Verfahren, umfassend die Schritte:
(i) Umsetzen von Ethylen mit Fluoralkyliodid der Formel Rf(CF₂CF₂)*ₙ*I (1), um ein Ethylenaddukt der Formel Rf(CF₂CF₂)*ₙ*CH₂CH₂I (2) herzustellen;
(ii) Umsetzen des erhaltenen Ethylenaddukts der Formel (2) mit mindestens einer Verbindung, ausgewählt aus Lithiumhydroxid, Natriumhydroxid, Lithiummethoxid, Lithiumethoxid, Natriummethoxid und Natriumethoxid, in Gegenwart eines niederen Alkohols und in Gegenwart von ≤ 11,5 Volumenteilen Wasser pro 100 Volumenteilen Alkohol, wodurch durch Dehydroiodierung eine Olefinverbindung der Formel Rf(CF₂CF₂)*ₙ*CH=CH₂ (3) und ein Alkalimetalliodid hergestellt werden; und
(iii) Sammeln des Alkalimetalliodids;
worin in den Formeln (1)-(3) Rf für C₁₋₁₀-Fluoralkyl steht und n eine ganze Zahl von ≥ 1 ist.

2. Verfahren gemäß Anspruch 1, wobei das Fluoralkyliodid der Formel (1) acht oder mehr Kohlenstoffatome aufweist.

3. Verfahren gemäß Anspruch 1 oder 2, wobei der in Schritt (ii) verwendete niedere Alkohol mindestens eines von Methanol und Ethanol ist, das Alkalimetallhydroxid Natriumhydroxid ist und das Alkalimetallalkoxid mindestens eines von Natriummethoxid und ist Natriumethoxid ist.

4. Verfahren gemäß einem der Ansprüche 1 bis 3, wobei das erhaltene Alkalimetalliodid ferner einer Iodbildungsbehandlung unterzogen wird, um festes Iod abzutrennen und das feste Iod zu sammeln.

5. Verfahren gemäß Anspruch 4, umfassend die Schritte:
(a) Herstellen einer wässrigen Lösung des Alkalimetalliodids, das gemäß einem der Ansprüche 1 bis 3 erhalten wurde;
(b) Behandeln der Lösung mit Chlor, um Iod abzutrennen, wodurch eine Iodaufschlämmung erhalten wird;
(c) Schmelzen von Iod in der Aufschlämmung durch Erwärmen, um eine Flüssig-Flüssig-Trennung zu induzieren, wodurch zwei Schichten aus Wasser und geschmolzenem Iod gebildet werden;
(e) Verfestigen des geschmolzenen Iods durch Abkühlen; und
(f) Sammeln des festen Iods.

6. Verfahren gemäß Anspruch 5, ferner umfassend zwischen den Schritten (c) und (e) den Schritt:
(d) Waschen des geschmolzenen Iods mit Wasser, um festes Iod zu erhalten, und Schmelzen des festen Iods durch erneutes Erwärmen, um eine Flüssig-Flüssig-Trennung zu induzieren, wodurch zwei Schichten aus Wasser und geschmolzenem Iod gebildet werden.

## Revendications

1. Procédé comprenant les étapes consistant à :
(i) faire réagir de l'éthylène avec l'iodure de fluoroalkyle de formule Rf(CF₂CF₂)*ₙ*I (1) pour préparer un adduit d'éthylène de formule Rf(CF₂CF₂)*ₙ*CH₂CH₂I (2) ;
(ii) faire réagir l'adduit d'éthylène obtenu de formule (2) avec au moins un composé choisi parmi l'hydroxyde de lithium, l'hydroxyde de sodium, le méthoxyde de lithium, l'éthoxyde de lithium, le méthoxyde de sodium et l'éthoxyde de sodium, en présence d'un alcool inférieur et en présence de ≤ 11,5 parties en volume d'eau pour 100 parties en volume d'alcool, en préparant ainsi, par déshydroiodation, un composé d'oléfine de formule Rf(CF₂CF₂)*ₙ*CH=CH₂ (3) et un iodure de métal alcalin ; et
(iii) collecter l'iodure de métal alcalin ;
dans lequel dans les formules (1) à (3), Rf est un fluoroalkyle en C₁ à C₁₀ et *n* est un entier ≥ 1.

2. Procédé selon la revendication 1, dans lequel l'iodure de fluoroalkyle de formule (1) a huit atomes de carbone ou plus.

3. Procédé selon la revendication 1 ou 2, dans lequel l'alcool inférieur utilisé dans l'étape (ii) est au moins un parmi le méthanol et l'éthanol, l'hydroxyde de métal alcalin est l'hydroxyde de sodium, et l'alcoxyde de métal alcalin est au moins l'un du méthoxyde de sodium et de l'éthoxyde de sodium.

4. Procédé selon l'une quelconque des revendications 1 à 3, dans lequel l'iodure de métal alcalin obtenu est en outre soumis à un traitement de formation d'iode pour préparer de l'iode solide, et à une collecte de l'iode solide.

5. Procédé selon la revendication 4, qui comprend les étapes consistant à :
(a) préparer une solution aqueuse de l'iodure de métal alcalin obtenu dans le procédé de l'une quelconque des revendications 1 à 3 ;
(b) traiter la solution avec du chlore pour séparer l'iode, en obtenant ainsi une barbotine d'iode ;
(c) faire fondre l'iode dans la barbotine par chauffage pour induire une séparation liquide-liquide pour former ainsi deux couches d'eau et d'iode fondu ;
(e) solidifier l'iode fondu par refroidissement ; et
(f) collecter l'iode solide.

6. Procédé selon la revendication 5, qui comprend en outre entre les étapes (c) et (e) une étape consistant à :
(d) laver l'iode fondu avec de l'eau pour donner de l'iode solide et faire fondre l'iode solide par réchauffement pour induire une séparation liquide-liquide pour ainsi former deux couches d'eau et d'iode fondu.
